# EUROPEAN PATENT SPECIFICATION

(11) **EP 3 449 913 B1**
(45) Date of publication and mention of the grant of the patent: **26.04.2023**
(21) Application number: 18766653.2
(22) Date of filing: 14.03.2018
(51) Int. Cl.: A61L 27/26, A61L 27/52, A61L 27/54, A61L 27/56, A61P 25/00, A61K 31/728, A61K 38/18, A61K 38/36, A61K 38/39, A61L 27/20, A61L 27/22

(54) **HYDROGEL PATCH**
HYDROGELPFLASTER
TIMBRE D'HYDROGEL

(30) Priority: 14.03.2017 KR 20170032019
(43) Date of publication of application: 06.03.2019
(73) Proprietor: Supine Therapeutics Co., Ltd., Ulsan 44919 (KR)
(72) Inventor: KIM, Jeong Beom, Ulju-gun Ulsan 44919 (KR); NAM, Dong Gyu, Ulju-gun, Ulsan 44919 (KR)
(74) Representative: Mewburn Ellis LLP
(86) International application number: PCT/KR2018/003011
(87) International publication number: WO 2018/169314

(56) References cited:
- KR-A- 20110 002 741
- US-A1- 2006 233 850
- US-A1- 2007 010 831
- US-A1- 2012 282 324
- ARULMOLI, J. et al.: "Combination Scaffolds of Salmon Fibrin, Hyaluronic Acid, and Laminin for Human Neural Stem Cell and Vascular Tissue Engineering", Acta Biomaterialia, vol. 43, 2016, pages 122-138, XP055548151,
- PARK, J. et al.: "Nerve Regeneration Following Spinal Cord Injury Using Matrix Metalloproteinase-sensitive, Hyaluronic Acid-based Biomimetic Hydrogel Scaffold Containing Brain-derived Neurotrophic Factor", journal of Biomedical Materials Research Part A, vol. 93, 2010, pages 1091-1099, XP055560185,
- LEE, F. et al.: "Formation and Stability of Interpenetrating Polymer Network Hydrogels Consisting of Fibrin and Hyaluronic Acid for Tissue Engineering", Acta Biomaterialia, vol. 9, 2013, pages 5143-5152, XP055450790,

## Description

### TECHNICAL FIELD

The present disclosure relates to a hydrogel patch, a method of producing the same, and a composition for the treatment of spinal cord injury including the same.

### BACKGROUND ART

Injury of the central nervous system (spinal cord) involves psychological disturbances as well as medical disorders such as motor and urinary disorders. In the treatment of spinal cord injury, surgical treatment, steroid-based medications such as methylprednisolone, or rehabilitation therapy is currently being used to alleviate nerve injury. However, these methods are only remedies which alleviate disorders caused by nerve damage, and to this day no fundamental spinal cord nerve regeneration therapy exists.

To induce regeneration of injured spinal cord nerves, a method of inducing a biological nerve regeneration environment in a nerve injury site, and cell therapies that involve administering of neural stem cells, spindle progenitor precursor cells, mesenchymal stem cells, Schwann cells or nerve epithelial cells are being developed. For the development of cell therapeutic agents used in the above cell therapies, adult stem cells, embryonic stem cells, or induced pluripotent stem cells are mainly used.

However, adult stem cells have problems of scarcity, limited differentiation ability, and immune rejection due to the use of foreign cells. In addition, embryonic stem cells have ethical problems and immune rejection due to the use of foreign cells. Embryonic stem cells and induced pluripotent stem cells commonly have carcinogenic potential.

In the case of spinal cord injury, 48 hours after injury, which is the acute period of spinal cord injury, is known to be appropriate for the treatment of spinal cord injury. In the case of a cell therapeutic agent, neuroregenerative cells such as autologous neurons and oligodendrocyte progenitor cells are necessary to avoid immunity rejection. However, during the acute period, it is impossible to collect cells from patients suffering from acute spinal cord injury and obtain such a cell population as being suitable for a cell therapeutic agent. In addition, although induced pluripotent stem cells prepared from a patient's somatic cells or directly crossed differentiated cells may meet the cell population required for the preparation of cell therapeutic agents due to their self-regenerating ability, the production time and the time period for differentiation into target cells are not suitable for treatment during the acute period. Accordingly, it is impossible to carry out the treatment during the acute period which is the appropriate time period for the treatment of spinal cord injury.

US 2012/282324 describes a method of treating a spinal cord injury by topically delivering to the injury site an amount of a growth factor effective to treat the spinal cord injury. This active agent is incorporated in a hydrogel which can comprise e.g. a combination of hyaluronic acid, collagen and laminin, or hyaluronic acid, gelatin and laminin.

Therefore, there is a need to develop a spinal cord nerve regeneration technique that minimizes spinal cord injury and induces regeneration of injured spinal cord nerves in a spinal cord in a non-invasive manner at the appropriate time period.

### SOLUTION TO PROBLEM

The scope of this invention is defined by the claims. Any "embodiment" or "example" which is disclosed in the description but is not covered by the claims should be considered as presented for illustrative purpose only. Embodiments in the description relating to methods of treatment are not covered by the claims.

The term "treatment" refers to or includes the alleviation, inhibition of the progress or prevention of a disease, disorder or condition, or one or more symptoms thereof, and the term "pharmaceutically effective amount" refers to any amount of a composition used in the practice of the present disclosure provided herein sufficient to alleviate, inhibit of the progress of, or prevent the disease, disorder or condition, or one or more symptoms thereof.

The terms "administering", "applying", "introducing", and "transplanted" are used interchangeably and refer to the placement of a patch or composition into a subject according to a method or pathway that results in at least partial localization of the patch or composition.

The term "patch" used herein may refer to an element having a certain shape and being applied, attached, or brought into contact a target site.

In one embodiment, the hydrogel patch or composition may have an intermediate property between solid and liquid. The hydrogel patch or composition may be amorphous, spherical, hemispherical, discular, or cylindrical. For example, the diameter of the hydrogel patch may be in the range of 0.05 mm to 10 cm, 0.1 mm to 5 cm, 0.1 mm to 3 cm, or 0.2 mm to 1.5 cm, and the hydrogel patch may be provided in such sizes or shapes. In addition, the hydrogel patch may be modified to conform to the shape of the injured site by applying, implanting, attaching, or contacting the target area (for example, an injured tissue site).

In one or more embodiments, the composition may be a solid (including powder), semi-solid, or liquid. In one embodiment, the hydrogel patch or composition may reversibly undergo phase-transition (for example, depending on the temperature) among a solid (including powder) state, a semi-solid state, or a liquid state. Since the hydrogel patch reversibly undergoes phase-transition depending on the surrounding conditions such as temperature conditions, the hydrogel patch according to an embodiment may be produced and provided in a solid state (including powder) or in a liquid state, and then, before, on, or after administration to a target site, and the hydrogel patch may be changed into a hydrogel state before use. For example, the hydrogel patch may be provided in the form of sol including fibrinogen, laminin, and a hyaluronic acid. In this case, due to the use of a material (for example, thrombin) that is capable of changing fibrinogen into fibrin, a user may manufacture a hydrogel patch according to an embodiment. Accordingly, the hydrogel patch according to an embodiment may be provided in the form of composition including fibrin and/or fibrinogen, laminin, and a hyaluronic acid, for example, in the form of a prodrug of the solid (powder), liquid (sol), or semi-solid composition. The composition provided in the form of the prodrug may be manufactured or modified in the hydrogel patch before acting. In addition, the hydrogel patch according to an embodiment may further include thrombin, or thrombin may be provided together as a kit.

In one or more embodiments, the hydrogel patch or composition may be porous. For example, the surface of the hydrogel patch may have a porosity (micropores). Without being limited to any particular theory, the hydrogel patch according to an embodiment may enhance the interaction between active materials due to their porosity.

The hydrogel patch or composition may be used in a spinal cord non-invasive manner on the patient's SCI site. In this specification, the non-invasive method or manner is distinguished from a cellular therapeutic agent or drug administration method which uses a spinal cord-invasive manner using a needle, and the hydrogel patch or composition may be transplanted on, applied on, attached on, or brought into contact a target site without any other means (for example, syringe). Unlike a spinal cord-invasive method in which a therapeutic composition is injected into a spinal cord in an invasive manner by using, for example, a needle, thereby causing SCI in the injection site, according to the spinal cord non-invasive method, a composition is delivered to an injured site by applying or transplanting without SCI caused by the injection of the composition through the needle. In addition, the hydrogel patch or composition may prevent secondary injury of the spinal cord caused by the invasive method. In one embodiment, the hydrogel patch may be biodegraded *in vivo* after a certain period of time.

According to the claimed invention, the hydrogel patch or composition includes fibrin and/or fibrinogen. A final pharmacological substance acting *in vivo* includes fibrin, but fibrinogen may be used instead of fibrin as the form of a prodrug. Accordingly, an aspect of the present disclosure provides a prodrug including fibrinogen, laminin, and a hyaluronic acid. Fibrin or fibrinogen may be included in an amount of 0.5 mg/ml to 20 mg/ml, 0.8 mg/ml to 16 mg/ml, 0.8 mg/ml to 12 mg/ml, 1.0 mg/ml to 10 mg/ml, 2 mg/ml to 8 mg/ml, or 4 mg/ml to 8 mg/ml.

Fibrinogen glycoprotein is a hexamer including soluble α, β, and γ subunits produced in liver hepatocytes. Fibrinogen reacts with thrombin enzyme and may undergo phase-transition from soluble to insoluble fibrin polymer fibers.

Thrombin enzyme is a serine protease, which is an enzyme that transforms a soluble fibrinogen into an insoluble fibrin. Thrombin may change fibrinogen to fibrin, thereby gelling the sol-phase hydrogel.

The term "laminin" used herein refers to an extracellular matrix protein constituting a basal lamina, which may denote a heterotrimeric protein consisting of α, β, and γ subunits. Thus, laminin may include a laminin full-length protein and a laminin-derived peptide or protein. For example, laminin may be laminin-1, laminin-2, laminin-3, laminin-4, laminin-5A, laminin-5B, laminin-6, laminin-7, laminin-8, laminin-9, laminin-10, laminin-11, laminin-12, laminin-14, or laminin-15. In one embodiment, the laminin-derived peptide may be an α chain, a γ chain, or a β chain. Laminin is included in the concentration of 2 µg/ml to 80 µg/ml, 5 µg/ml to 50 µg/ml, 5 µg/ml to 25 µg/ml, 8 µg/ml to 20 µg/ml, or 8 µg/ml to 15 µg/ml. Hyaluronic acid glycosaminoglycan is a polysaccharide having a disaccharide bond in which D-glucuronic acid and N-acetyl-D-glucosamine have glycosidic bonds with changes of β- (1 → 4) and β- (1 → 3), and a molecular weight thereof varies depending on the length of the disaccharide bond. In one embodiment, the molecular weight of a hyaluronic acid may be in a range of 5,000 Da to 20,000,000 Da. In one embodiment, the molecular weight of a hyaluronic acid may be in the range of 0.5 to 4.0 × 10⁶ Da, 1.0 to 2.0 × 10⁶ Da, or 1.5 to 1.8 × 10⁶ Da. Hyaluronic acid is

included in a range of 10 µg/ml to 5 mg/ml, 50 µg/ml to 5 mg/ml, 100 µg/ml to 3 mg/ml, 100 µg/ml to 1 mg/ml, 200 µg/ml to 1 mg/ml, or 300 µg/ml to 800 µg/ml. In one embodiment, the hyaluronic acid may be provided in the form of a salt, a pharmaceutically acceptable salt, for example, a sodium salt, a potassium salt, a calcium salt, or a magnesium salt.

In one embodiment, the hydrogel patch or composition may further include a cell growth factor. The cell growth factor may be a neuronal cell growth factor, a vascular endothelial cell growth factor, a fibroblast growth factor, a bone morphogenetic protein, an epidermal growth factor, a hepatocyte growth factor, a transformational growth factor, or a combination thereof. In one embodiment, the cell growth factor may include a placenta growth factor, a macrophage colony stimulating factor, a granulocyte macrophage colony stimulating factor, a neurofilin, a fibroblast growth factor(FGF)-1, FGF-2 (bFGF), FGF-3, FGF-4, FGF-5, FGF-6, Erythropoietin, BMP-2, BMP-4, BMP-7, TGF-beta, IGF-1, oteopontin, plastrophin, activin, Endocellin-1, or a combination thereof. The neuronal growth factor may include at least one selected from a brain-derived neurotrophic factor (BDNF), a glial cell-derived neurotrophic factor (GDNF), a ciliary neurotrophic factor (CNTF), a basic fibroblast growth factor (bFGF), a cyclic adenocyne monophosphate (cAMP), neurotropin (NT), a neurotropin-3 (NT3), a neurotropin-4 (NT4), triiodo-L-thyronine (T3), sonic hedgehog (SHH), and a platelet-derived growth factor (PDGF). The vascular endothelial growth factor may include vascular endothelial growth factor (VEGF) -A, VEGF-A, VEGF-B, VEGF-C, VEGF-D, or VEGF-E. The concentration of the cell growth factor included in the hydrogel patch or composition may vary according to the cell growth factor, and may be in a range of 1 ng ml to 1,000 ng / ml or 0.1 µM to 100 µM. The cell growth factor may increase the injured tissue recovery effects of the hydrogel path or composition including fibrin, laminin, and a hyaluronic acid.

The brain-derived neurotrophic factor (BDNF) protein is encoded by a BDNF gene. The BDNF protein is known to help the survival of neurons in central nervous system and the differentiation and growth of new neurons.

The glial cell line-derived neurotrophic factor (GDNF) protein is encoded by a GDNF gene. The GDNF protein is known to help the survival and differentiation of dopaminergic neurons and motor neurons among neurons in the central nervous system.

The ciliary neurotrophic factor (CNTF) protein is encoded by a CNTF gene. The CNTF protein is known to promote the production of neurotransmitters and help the survival of neurons and oligodendrocytes.

The neurotrophin-3 (NT-3) protein is encoded by an NT-3 gene. The NT-3 protein is known to help the survival of neurons in central nervous system and the differentiation and growth of new neurons.

The cyclic adenosine monophosphate (cAMP) protein is a protein that is derived from adenosine triphosphate (ATP) by the action of adenylate cyclase, and acts as a second messenger of cells. The cyclic adenosine monophosphate (cAMP) protein is known to be involved in the overall regulation of neurotransmitters, such as the production, storage and distribution of neurotransmitters, to be helpful in the survival and differentiation of neurons, and to act as a barrier of vascular endothelial cells, and to help proliferation and the production of nitric oxide.

The sonic hedgehog (SHH) protein is encoded by a SHH gene. SHH plays a role in the hedgehog signaling pathway, and in the case of the nervous system, is known to act in motor neuron differentiation.

Triiodothyronine (T3) hormone is a kind of thyroid hormone and affects most physiological actions *in vivo.* In the nervous system, triiodothyronine (T3) hormone is to inhibit the differentiation of oligodendrocyte progenitor cells and promote the differentiation into oligodendrocyte.

The subtype of platelet-derived growth factor (PDGF) may be -AA,-BB,-AB,-CC, and -DD, and in the case of homodimer of PDGFA subunit encoded by platelet-derived growth factor subunit A (PDGFA) gene, the subtype thereof may be PDGF-AA, and each subtype binds to a different receptor (PDGFR) of PDGF. Platelet-derived growth factor-AA (PDGF-AA) is a dimer glycoprotein, and is known to maintain the proliferation of oligodendrocyte progenitor cells together with fibroblast growth factor (FGF), which activates the PDGF receptor of oligodendrocyte progenitor cells.

Vascular endothelial growth factor (VEGF) protein is a type of vascular permeability factor (VPF) that acts in angiogenesis and vascularization. VEGF is known to act in the formation of new blood vessels in embryonic development and scar tissues, and to help the survival of neurons by preventing the death of neurons due to toxicity and stress.

In one or more embodiments, the hydrogel patch or composition may include or may not substantially include collagen. Without being limited to any particular theory, the collagen may be included or not substantially included as a component of the composition. According to an aspect, the absence of the collagen may be advantageous compared to the presence thereof.

According to the claimed invention, the hydrogel patch or composition does not include cells. The hydrogel patch or composition according to the claimed invention is substantially different from a cell therapeutic agent used for the regeneration of injured tissues due to the absence of cells therein.

The collagen protein is classified into Type I, II, III, IV, and V, and Type I collagen is the most abundant in the body. Collagen has a triple helix structure of α1 and α2.

According to the claimed invention, the hydrogel patch or composition includes fibrin and/or fibrinogen, laminin, and a hyaluronic acid or a salt thereof. In addition, the hydrogel patch or composition may further include at least one of the components described in the present specification. For example, the hydrogel patch or composition may include fibrin and/or fibrinogen, laminin, a hyaluronic acid or a salt thereof, optionally thrombin, optionally collagen; and optionally a cell growth factor.

In one embodiment, the hydrogel patch or composition may enhance cell adhesion. Accordingly, disclosed herein, but not claimed, is a method of enhancing adhesion of a subject's cell (for example, a neuron), the method including administering the hydrogel patch or composition to the subject.

In one or more embodiments, the hydrogel patch or composition may increase the differentiation of neural stem cells into neurons. Accordingly, disclosed herein, but not claimed, is a method of increasing the differentiation of neural stem cells of a subject into neurons, the method including administering the hydrogel patch or composition to the subject.

In one or more embodiments, the hydrogel patch or composition may inhibit nerve injury by reducing the inflammatory response, or to promote regeneration of the motor neurons having myelin sheath by helping the regeneration of motor neurons and oligodendrocytes.

In one or more embodiments, the hydrogel patch or composition may induce regeneration of nerves injured due to external injury, such as SCI, and nerves injured due to neural degeneration such as a stroke. Accordingly, disclosed herein, but not claimed, is a method of inhibiting neuron injury of a subject, inducing nerve regeneration, or regenerating motor neurons, the method including administering the hydrogel patch or composition to the subject.

In one or more embodiments, the hydrogel patch or composition may inhibit a secondary injury to a spinal cord caused by the treatment of SCI.

Thus, the hydrogel patch or composition according to the claimed invention is for use in regenerating or covering an injured spinal cord tissue. a spinal cord.

The term "spinal cord injury (SCI)" used herein refers to an injury caused by spinal cord compression or dislocation. The SCI may include external SCI, spinal degenerative diseases (such as spondylosis), spinal inflammatory diseases (spondylitis, chronic rheumatoid arthritis, etc.), tumor (spinal cord tumor, spinal tumor, etc.), vascular diseases (spinal cord bleeding, stroke, spinal cord paralysis due to extramedullary vascular disorder, etc.), myelitis (ararchnoiditis, viral myelitis, bacterial myelitis, etc.), multiple sclerosis, amyotrophic lateral sclerosis, or the like. The hydrogel patch or composition according to an embodiment may have therapeutic effects not only on acute SCI but also on chronic SCI. Accordingly, the SCI may be a chronic SCI.

The dosage of the composition according to an embodiment may be in a range of 0.01 mg to 10,000 mg, 0.1 mg to 1000 mg, 1 mg to 100 mg, 0.01 mg to 1000 mg, 0.01 mg to 100 mg, 0.01 mg to 10 mg, or 0.01 mg to 1 mg. However, the dosage may be variously prescribed in consideration of factors such as the formulation method, the administration method, and the age, body weight, sex, and pathological condition of the patient, food, administration time, administration route, excretion rate, and responsiveness of the patient, These factors may be taken into consideration to adjust the dosage appropriately by one of ordinary skill in the art. The dosage may be administered once or twice or more within the scope of clinically acceptable side effects, and the administration site may be one or two or more. For animals other than humans, the same dosage as used for the human per kg, or the amount of the dose converted in terms of the volume ratio (for example, average value) of the organ of the target animal to the human organ (heart, etc.) may be used for the administration. Available routes of administration include oral, sublingual, parenteral (e.g. subcutaneous, intramuscular, intraarterial, intraperitoneal, intradural, or intravenous), rectal, and topical routes (including transdermal), inhalation, and injection, or insertion of a portable device or substance. An animal to be treated according to an embodiment includes humans and other mammals, and examples thereof are humans, monkeys, mice, rats, rabbits, sheep, cows, dogs, horses, pigs and the like.

The pharmaceutical composition according to an embodiment may include a pharmaceutically acceptable carrier and/or an additive. Examples thereof include sterilized water, physiological saline, buffers for common use (phosphoric acid, citric acid and other organic acids), stabilizers, salts, antioxidants (such as ascorbic acid), surfactants, suspending agents, isotonic agents, and preservatives. For topical administration, organic materials such as biopolymers, inorganic materials such as hydroxyapatite, for example, collagen matrix, polylactic acid polymer or copolymer, polyethylene glycol polymer or copolymer and chemical derivatives thereof may be included.

The pharmaceutical composition according to an embodiment may contain, depending on the administration method or formulation, if needed, various additives such as suspending agents, solubilizers, stabilizers, isotonizing agents, preservatives, adsorption inhibitors, surfactants, diluents, excipients, pH adjusters, analgesic agents, buffers, reducing agents, antioxidants, and the like. Pharmaceutically acceptable carriers and formulations suitable for the present disclosure, including those described above, are described in detail in [Remington's Pharmaceutical Sciences, 19th ed., 1995.] The pharmaceutical composition according to an embodiment may be formulated into a unit dosage form or placed in a multi-dose container by using a pharmaceutically acceptable carrier and/or excipient according to any method that is easily carried out by one of ordinary skill in the art. In this regard, the formulations may be in the form of solutions, suspensions or emulsions in an oil or aqueous medium, or in the form of powders, granules, tablets or capsules.

Also described herein is a method of preparing a hydrogel patch, the method including adding thrombin to a sol-phase composition including fibrinogen, laminin or laminin-derived peptides; and a hyaluronic acid or a pharmaceutically acceptable salt thereof.

The method may further include adding a cell growth factor to the composition in the sol state.

The method may also include a gelling by adding thrombin, followed by a second gelation by adding thrombin thereto. The gelation may be performed at a temperature of 10 °C to 40 °C for 5 minutes to 3 hours. In addition, thrombin may be added at a concentration of about 1 Ulml to about 10 U/ml. The hydrogel patch may be manufactured in various shapes or sizes depending on the shape of a frame.

The method may also include low-temperature preserving or cryopreserving the hydrogel patch at a temperature of 4 °C to -210 °C in a solution. The solution may include dimethyl sulfoxide (DMSO), and may not be limited as long as it does not substantially change the chemical or physical properties of the hydrogel patch. The hydrogel patch does not substantially change its shape or activity even when it is low-temperature preserved or cryopreserved.

The hydrogel patch, fibrin and/or fibrinogen, laminin, and hyaluronic acid, or the cell growth factors are as described above.

Also described herein is a method of low-temperature preserving or cryopreserving the hydrogel patch in a solution (for example, DMSO) at a temperature of 4 °C to -210 °C in solution.

Hydrogel patches preserved at low temperature or cryopreserved in the above manner may be used immediately for the treatment of patients suffering from spinal cord injuries during the acute period, which is the optimal treatment timing for SCI. Even when the hydrogel patches that have been preserved at low temperature or cryopreserved are melted at room temperature, the hydrogel patches may not experience morphological changes and may retain the therapeutic effect on the SCI.

### BRIEF DESCRIPTION OF DRAWINGS

FIG. 1 is a view illustrating a hydrogel patch preparation method according to an embodiment and a hydrogel patch having a size of 0.3 mm to 0.8 mm.
FIG. 2 shows photographs illustrating a hydrogel patch formulation (liquid, gel, powder) (top panels, left to right) and gelation (bottom panels) of a powder formulation according to an embodiment.
FIG. 3 shows scanning electron microscope (SEM) images of a cross-section and a surface of a hydrogel patch according to an embodiment before and after cryopreservation thereof.
FIG. 4 shows effects of the composition (fibrin, hyaluronic acid, and laminin) of a hydrogel patch according to an embodiment on mouse neural stem cell differentiation *in vitro,* as confirmed by optical microscopy and immunofluorescence staining, compared with when the composition is not used (bare); the scale bars represent 100 µm and 50 µm, respectively.
FIG. 5 shows immunofluorescence staining images of distribution of neuronal (Tuj1) and astrocytic (GFAP) differentiation, showing effects of a hydrogel patch according to an embodiment on mouse neural stem cell differentiation *in vitro,* when the hydrogel patch includes each component alone and when the hydrogel patch includes a combination of the two components; the scale bar represents 50 µm.
FIG. 6 shows a neuronal (Tuj1) differentiation distribution graph which is used to quantify effects of a hydrogel patch according to an embodiment on mouse neural stem cell differentiation *in vitro,* when the hydrogel patch includes each component alone and when the hydrogel patch includes a combination of the two components; * p< 0.05, ** p< 0.005, and *** p< 0.0005.
FIG. 7 shows a neuronal (Tuj1) differentiation distribution graph which is used to quantify effects of a hydrogel patch according to an embodiment on mouse neural stem cell differentiation *in vitro,* when a growth factor is added to the hydrogel patch including each component alone and the hydrogel patch including a combination of the two components; * p< 0.05, ** p< 0.005, and *** p< 0.0005.
FIG. 8 shows a neuronal (Tuj1) differentiation distribution graph which is used to quantify effects of a hydrogel patch according to an embodiment on mouse neural stem cell differentiation *in vitro,* when a growth factor (FGF, BDNF, GDNF, cAMP, NT3, PDGF-AA, SHH, T3, or VEGF) is added to the hydrogel patch; * p< 0.05, ** p< 0.005, and *** p< 0.0005.
FIG. 9 confirms biodegradation of a hydrogel patch according to an embodiment when the hydrogel patch is transplanted *in vivo.*
FIG. 10 shows images illustrating a process of transplanting a hydrogel patch according to an embodiment into an injured spinal cord.
FIG. 11 shows images of a spinal cord injury (SCI) animal model for observation of the movement of hind legs thereof, one week after SCI.
FIG. 12 shows an image of an SCI animal model for observation of the movement of hind legs thereof, eight weeks after SCI, when PBS alone, without the hydrogel patch, is applied to the transplantation site.
FIG. 13 shows an image of an SCI animal model for observation of the movement of hind legs thereof, eight weeks after SCI, when hydrogel patch 2 (hydrogel patch 2 without GF) is transplanted.
FIG. 14 shows an image of an SCI animal model for observation of the movement of hind legs thereof, eight weeks after SCI, when hydrogel patch 5 (hydrogel patch 2 with GF) is transplanted.
FIG. 15 shows BBB scores obtained according to transplantation of a hydrogel patch according to an embodiment (hydrogel patch 3 with PBS, hydrogel patch 3, and hydrogel patch 6).
FIG. 16 shows BBB scores obtained according to transplantation of a hydrogel patch according to an embodiment (hydrogel patch 1, hydrogel patch 2, and hydrogel patch 3).
FIG. 17 shows BBB scores obtained according to transplantation of a hydrogel patch according to an embodiment (hydrogel patch 4, hydrogel patch 5, and hydrogel patch 6).
FIG. 18 shows BBB scores obtained according to transplantation of a hydrogel patch according to an embodiment (hydrogel patch 2 and hydrogel patch 5).
FIG. 19 is an image showing histological analysis results obtained according to transplantation of a hydrogel patch according to an embodiment.

### MODE OF DISCLOSURE

Hereinafter, the present disclosure will be described in detail by reference to Reference Examples and Examples. The following Reference Examples and Examples are illustrative of the present disclosure and are not to be construed as limiting the present disclosure.

### <Examples> Hydrogel blend and patch production

### 1-1. Hydrogel blend

20 mg/ml of fibrinogen (Sigma, F8630) was dissolved in DMEM/F12(1:1) (Gibco, 11330-057) culture at a temperature of 37 °C for 30 minutes, 5 mg/ml of a hyaluronic acid (Sigma, 53747) was dissolved in DMEM/F12(1:1) (Gibco, 11330-057) culture at a temperature of 4 °C for a day, and 200 U/ml of thrombin (Sigma, T4648) was dissolved in DMEM/F12(1:1) (Gibco, 11330-057) culture. Collagen (Corning, 354236) was neutralized and ionized in 10X Dulbecco's Phosphate-Buffered Saline (DPBS, Sigma, D5652-10L), deionized water, and 1N NaOH (Millipore, 1.00983.1011), and diluted until the final concentration thereof reached 3.00 mg/mL.

Then, the above components were blended to prepare a hydrogel in a sol state. The final concentrations of each component are as follows:
Fibrinogen (Sigma, F8630) 1 mg/ml, 5 mg/mL or 10 mg/ml;
Laminin (thermofisher, 23017-015) 5 µg/ml, 10 µg/ml or 50 µg/ml;
Hyaluronic acid (Sigma, 53747) 0.1 mg/ml, 0.5 mg/ml or 1 mg/ml; and
Collagen (Corning, 354236) 1.2 mg/ml.

### 1-2. Addition of neuron growth factor to hydrogel blend

The hydrogel in a sol state prepared according to Example 1-1 was mixed with the following neuron growth factors and/or vascular endothelial cell growth factors in a culture in which DMEM/F12 (Gibco 11330-057), including penicillin/streptomycin (Invitrogen, 15140-122), 2 mM L-Glutamine (invitrogen, 25030-081), N2 supplement (Gibco, 1750-048), and B27 supplement minus vitamin A (Gibco, 12587010), and neurobasal medium (Gibco, 21103049) were mixed at a ratio of 1:1:
recombinant Human BDNF (Peprotech, 450-02) 10 ng/ml, recombinant Human GDNF (Peprotech, 450-10) 10 ng/ml, recombinant Human NT-3 (Peprotech, 450-03), 5 ng/ml, db-cAMP (Sigma, D0260) 1 uM, T3 (Sigma, T6397) 60 ng/ml, recombinant Human sonic hedgehog (SHH; Peprotech, 100-45) 50 ng/ml, recombinant Human PDGF-AA (Peprotech, 100-13A-100), recombinant Human FGF-basic (Peprotech, 100-18B) 10 ng/ml, and recombinant Human VEGF165 (Peprotech, 100-20) 20 ng/ml.

### 1-3. Preparation of hydrogel patch

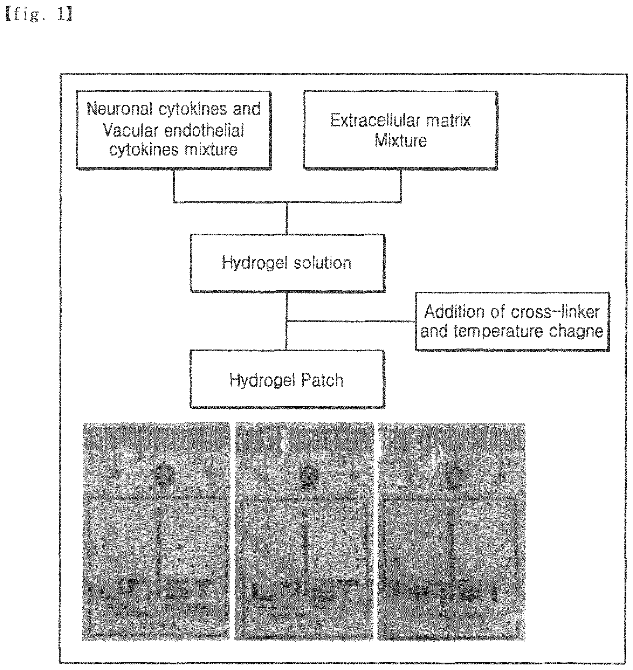

A hydrogel patch was prepared as illustrated in FIG. 1.

In detail, 5 U/ml of thrombin (Sigma, T4648) was added to the sol-phase hydrogel prepared according to Example 1-1 or 1-2 and incubated at 37 ° C for 1 hour for gelation. Then, a parafilm sterilized with ultraviolet light was punched remaining round pores having a size of 0.3 mm to 0.8 mm and the obtained structure was attached on a 10 cm Petri dish. Subsequently, 5 U/ml of thrombin was dispensed into the round pore, and 15 uL to 60 uL of hydrogel was dispensed thereon and the hydrogel and thrombin were mixed while suppressing the formation of bubbles, followed by gelling for about 2 minutes at room temperature, and then subjected to a secondary gelation process at 37 ° C for 1 hour. The gel-state hydrogel was separated from the paraffin structure to obtain patches of various sizes ranging from 0.3 mm to 0.8 mm. The prepared hydrogel patch was preserved in the culture including a neuron growth factor and a vascular endothelial growth factor prepared according to Example 1-2.

The hydrogel prepared by the above method is defined as a hydrogel patch, and types of the hydrogel patch prepared are summarized below.

In the following description, these hydrogel patches will be referred to as hydrogel patch 1 to 6. Hereby hydrogel patches 2, 3, 5 and 6 are according to the claimed invention, and hydrogel patches 1 and 4 are to be considered as reference examples.
Hydrogel patch 1: fibrin,
Hydrogel patch 2: fibrin + laminin + hyaluronic acid
Hydrogel patch 3: fibrin + laminin + hyaluronic acid + collagen
Hydrogel patch 4: fibrin + neuron growth factor and vascular endothelial growth factor (all growth factors listed in Example 1-2)
Hydrogel patch 5: fibrin + laminin + hyaluronic acid + neuron growth factor and vascular endothelial growth factor(all growth factors listed in Example 1-2)
Hydrogel patch 6: fibrin + laminin + hyaluronic acid + collagen + neuron growth factor and vascular endothelial growth factor (all growth factors listed in Example 1-2)

### 1-4. Formulation of hydrogel patch and possibility for use of cryopreservation

Hydrogel patches were prepared in various formulations, and it was confirmed whether they were able to be cryopreserved.

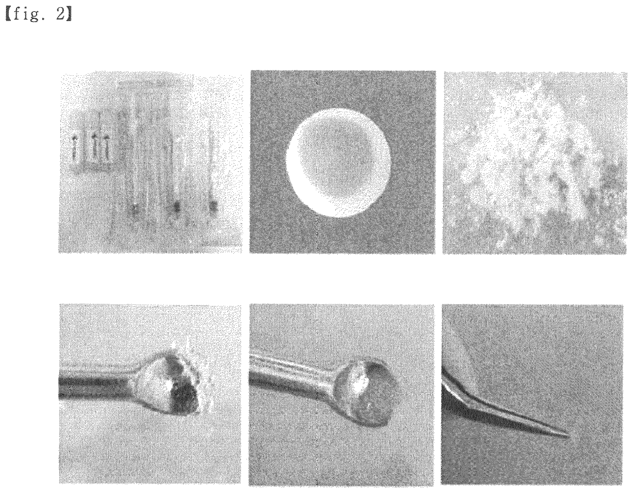

In detail, the reversible phase transition of hydrogel patch 2 prepared according to Example 1-3 was confirmed, and the results are shown in FIG. 2.

As shown in FIG. 2, hydrogel patch 2 was prepared in a liquid formulation in the same manner as in Example 1-1, and immersed in a vial. Thrombin was immersed in a syringe in the same manner as Example 1-3. In addition, hydrogel patch 2 was made into a solid formulation by freeze-drying (Scientific, F78) for 24 hours at -80 °C and 0.33 torr, and then crushed to prepare powder. In addition, a powder formulation was prepared. The above powder formulation was gelled by adding thrombin thereto in the same manner as in Example 1-3, thereby confirming that the powder formulation could return back in the form of gel.

This result shows that a hydrogel patch according to an embodiment exhibits reversible phase transition, and is able to be provided in various formulations such as solid, semi-solid, liquid or powder formulations.

In addition, it was confirmed whether cryopreservation was possible.

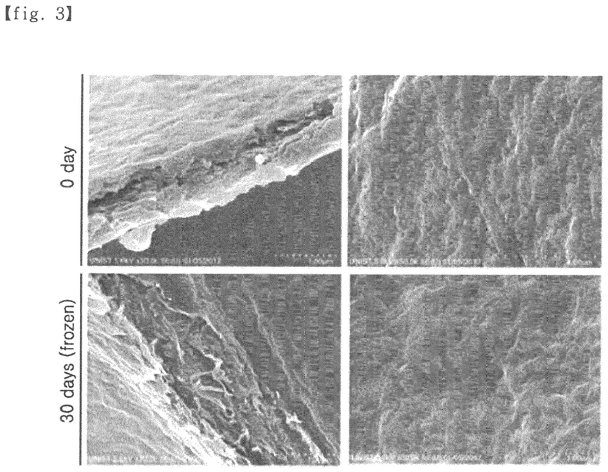

In detail, hydrogel patch 2 was added to 10% (v/v) DMSO prepared by adding dimethyl sulfoxide (DMSO, Sigma, D2650) to the culture prepared according to Example 1-2. Thereafter, the temperature was sequentially lowered to 4 °C (30 minutes), -20 °C (1 hour), and -80 °C, and the resultant was freeze-dried. The morphological analysis of the hydrogel patch before and after cryopreservation was carried out using a cold FE-SEM (Hitachi High-Technologies, S-4800), and the result is shown in FIG. 3.

As shown in FIG. 3, there was no morphological difference in the hydrogel patch before cryopreserve and the hydrogel patch that was thawed after one month of cryopreservation. Also, the surface of the hydrogel patch was found to be porous.

### <Experimental Example 1> Evaluation of neural stem cell differentiation in vitro

5-day-old C57BL/6 mice (C57BL/6N Japan SLC, Inc.) were sacrificed and brains thereof were collected, and mouse neural stem cells were primary cell cultured by a known method and cultured in a 100 mm dish (SARSTEDT, 831802).

A material according to an embodiment and a comparative material were separately applied on the mouse neural stem cells to confirm effects thereof on the differentiation of mouse neural stem cells.

Mouse neural stem cells were extracted and cultured by using a known method (Kim JB et al. Nat Protoc. 2009), and a hydrogel (fibrin 5 mg/mL; laminin 10 µg/ml; and a hyaluronic acid 0.5 mg/ml) prepared according to Example 1-2 was applied on the mouse neural stem cells having the population of 1×10⁴, followed by the addition of thrombin, thereby producing a hydrogel patch. Subsequently, the cells were cultured in a culture containing or not containing the neuron growth factor and vascular endothelial growth factor of Example 1-2. To select the optimal concentration, fibrin alone in an amount of 1 mg/ml, 5 mg/ml, or 10 mg/ml, laminin alone in an amount of 5 µg/ml, 10 µg/ml, or 50 µg/ml, and a hyaluronic acid alone in an amount of 0.1 mg/ml, 0.5 mg/ml, or 1 mg/ml, or laminin in an amount of 10 µg/ml, and a hyaluronic acid in an amount of 0.5 mg/ml were used as comparative materials.

Next, after 14 days of culture, immunofluorescence staining was performed. In detail, for immunocytochemistry, cells were fixed with 4% (w/v) paraformaldehyde in phosphate buffer solution (Wako, 163-20145) for 10 min at room temperature. The fixed cells were diluted 1X with 10X phosphate buffer saline (PBS, P2007), washed three times for 5 minutes at room temperature, and then permeabilized through 0.1% (v/v) triton X-100 (Sigma, T9284) for 10 minutes. After washing three times with 1X PBS for 5 minutes at room temperature, the cells were blocked with 4% (v/v) fetal bovine serum dissolved in 1X PBS for 1 hour at room temperature to inhibit nonspecific binding. Then, the cells were incubated at room temperature for 1 hour by using primary antibody anti-beta III tubulin(Tuj1) (1:400; Abeam) or anti-glial fibrillary acidic protein (GFAP) (1:400; Sigma), and washed three times for 10 minutes at room temperature by using 0.05% (v/v) Tween-20 (Sigma, P7949) (PBST) dissolved three times in 1X PBS. Subsequently, light was blocked with secondary fluorescent antibodies (alexa fluorophore-conjugated secondary antibodies 488 (1 :1000) or Alexa Fluor^{®}594 (1 :1000) and then, incubated at room temperature for 30 minutes. When double staining was required, additional blocking was performed at room temperature for 30 minutes before incubation with the other primary antibody. After washing three times for 10 minutes at room temperature with PBST, for cell nuclear staining, the cells were incubated with DAPI (1: 1000; Invitrogen) for 15 seconds, and washed three times for 10 minutes at room temperature by using PBST. Cells were stored in PBS for visualization using a fluorescence microscope.

Subsequently, neuron (Tuj1) and astrocytes (GFAP) were observed by using an inverted fluorescence microscope (Leica, DMI 3000B) with a digital monochrome camera attached (Leica, DFC345 FX). From the image observed with the inverted fluorescence microscope, the number of neurons or astrocytes was counted by using the imaged (National Institute of Health (http://rsb.info.nih.gov/ij) software to calculate the differentiation rate.

All statistical analysis were performed by using unpaired two-tailed Student's t-test. The significance was * P <0.05, ** P <0.005, or *** P <0.0005.

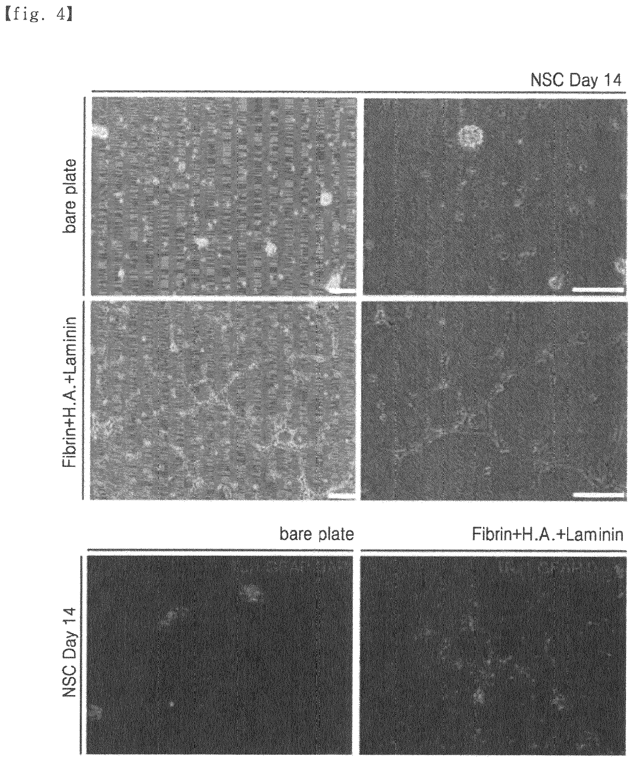

As shown in FIG. 4, when the hydrogel was applied on the neural stem cells according to an embodiment, it was found that the cell junction of the neural stem cells was improved as compared with the bare condition in which the hydrogel was not used.

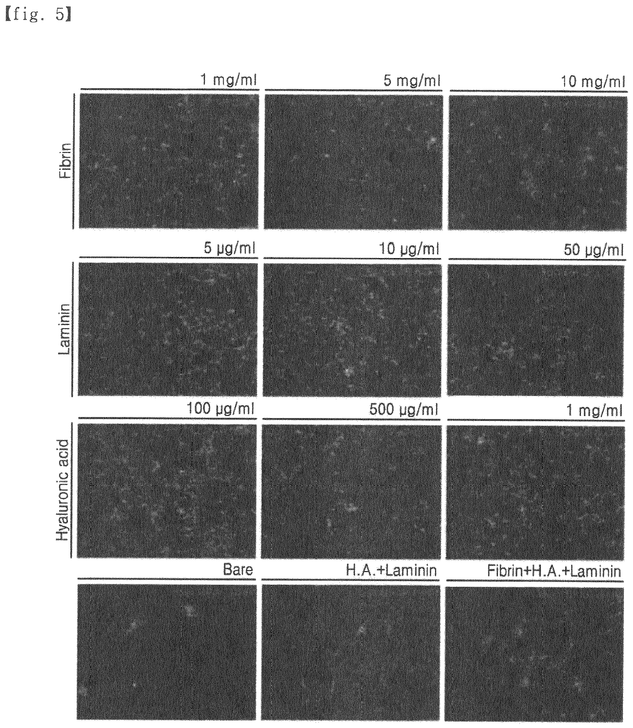

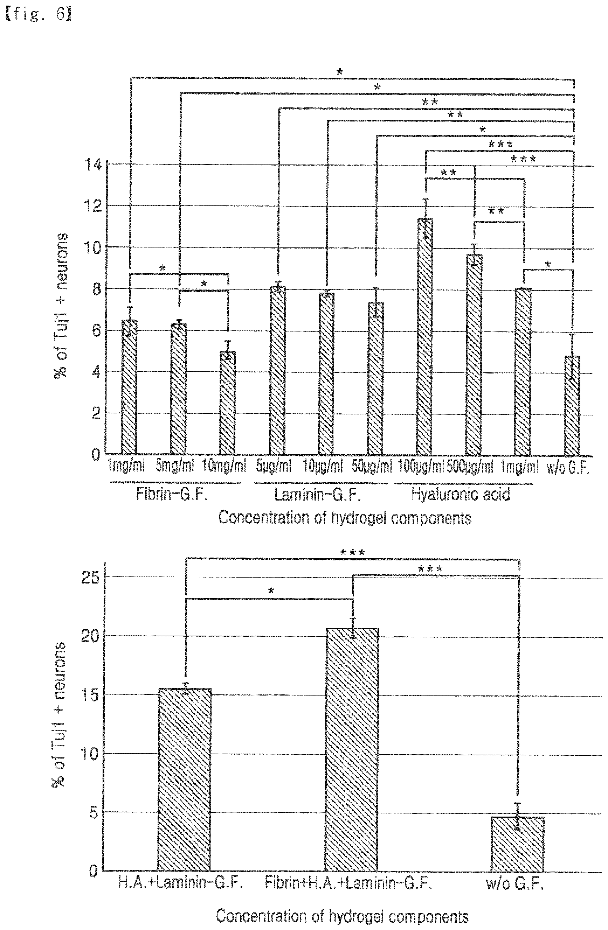

As shown in FIG. 5 and FIG. 6, in comparison with fibrin alone, laminin alone, a hyaluronic acid alone, and the combination of laminin and a hyaluronic acid, when the composition according to an embodiment (fibrin, laminin, and a hyaluronic acid) is applied, it can be seen that the differentiation of the neural stem cells into neurons occurred synergistically. In detail, 5% of neurons were present in the control group, and less than 12% of neurons were present in each component alone. However, in the case of the combination of a hyaluronic acid and laminin, about 15% of neurons are present, and when fibrin, which has no substantial effect on its own, was combined with a hyaluronic acid and laminin, it was found that at least 20% of neurons appeared. This suggests that the combination of a hyaluronic acid and laminin is effective in differentiating neurons, and in particular, the combination of the three compositions has a synergistic effect and markedly increases the differentiation into neurons.

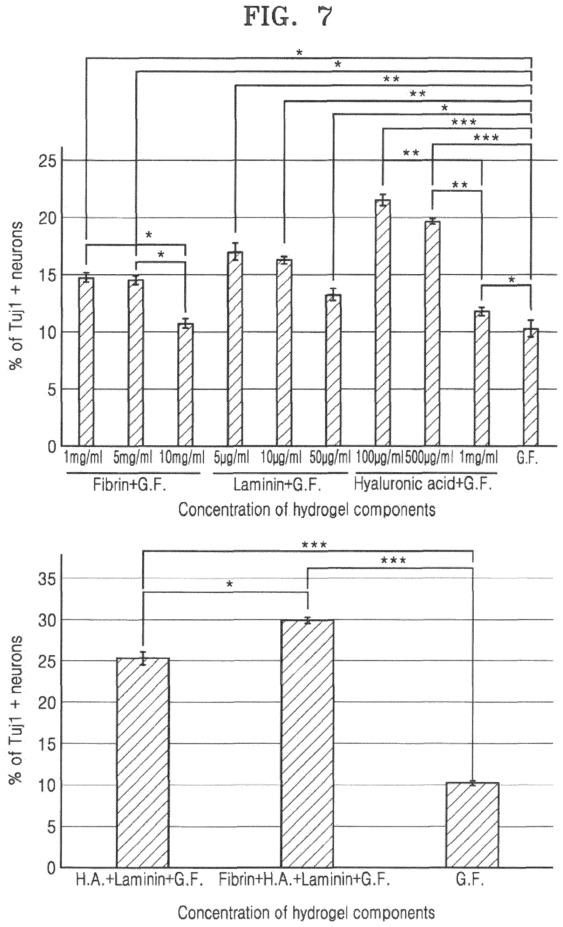

As illustrated in FIG. 7, when the growth factor was added, like the result illustrated in FIG. 6, in comparison with fibrin alone, laminin alone, a hyaluronic acid alone, and the combination of laminin and a hyaluronic acid, when the composition according to an embodiment (fibrin, laminin, and a hyaluronic acid) is applied, it can be seen that the differentiation of the neural stem cells into neurons occurred synergistically. In detail, although the growth factor itself increases the differentiation into neurons, the combination of the three compositions and the growth factor produces a synergistic effect, leading to about 30% of neurons. This result shows that the hydrogel patch or composition according to an embodiment shows a synergistic effect that is unpredictable in general.

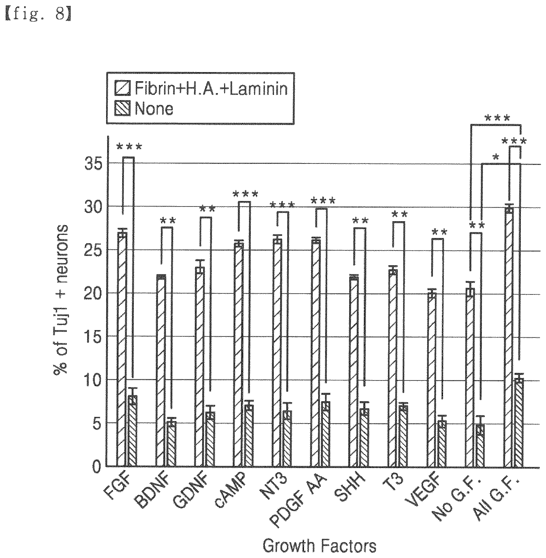

In addition, as illustrated in FIG. 8, each of the growth factors was added to the composition according to an embodiment composition, and as a result, it was confirmed that the neuron differentiation rate of mouse neural stem cells was improved by about 2.5 to 5 times.

As a result, it can be seen that the composition according to an embodiment of the present disclosure alone produces a synergistic effect of increasing the differentiation rate, and when combined with a growth factor, the combination produces a higher synergistic effect and the effects of growth factor on the cells are increased.

### <Experimental Example 2> Evaluation of biodegradability

To evaluate biodegradability of the hydrogel patch prepared according to Example 1, 6-week-old C57BL/6 mice (C57BL/6N Japan SLC, Inc.) was anesthetized with 25 mg/ml of Avertin anesthetic, which was prepared by dissolving 2,2,2-tribromoethanol (Sigma, T48402) in tert-amyl alcohol (Sigma, 152463). In this regard, the amount of anesthetic per mouse was 125 - 250 mg/kg body weight. Subsequently, hydrogel patch 5 was subcutaneously transplanted. Two weeks after the transplantation of the hydrogel patch, it was confirmed whether the subcutaneously transplanted hydrogel patch biodegraded.

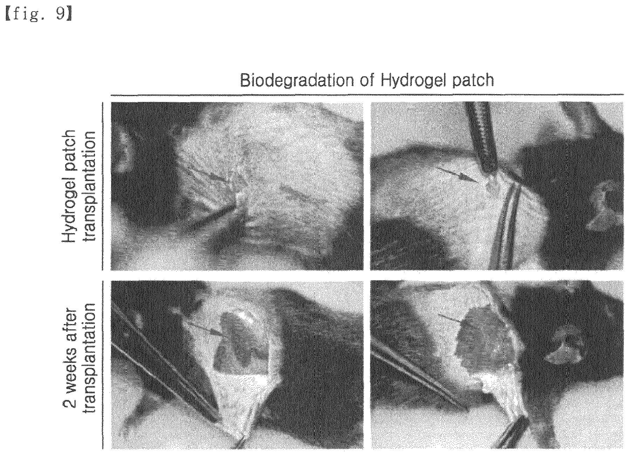

As a result, as illustrated in FIG. 9, it was confirmed that the hydrogel patch according to an embodiment was biodegraded *in vivo.*

### <Experimental Example 3> Spinal cord injury (SCI) treatment effects of hydrogel patch

### 4-1. Confirmation of treatment effects of hydrogel patch SCI by using SCI animal model

To confirm SCI regeneration effects of a hydrogel patch according to an embodiment, a gel patch was transplanted into a SCI animal model and then a behavioral test was performed thereon for 8 weeks.

An evaluation method of official small SCI animal model is to evaluate behavioral analysis of chronic SCI study, in which joints, hind legs, gait, harmony movement of forelegs and hind legs, the position of waist, support by soles, and the position of tails are observed and evaluated to identify the recovery of the behavioral ability. The obtained evaluation result of each animal, that is, the recovery ability was quantified at a scale of 0 to 21: the scale of a to 7 indicates an early stage in which the minimal behavioral ability was recovered, that is, the movement of the hind legs were hardly recovered, and the scale of 8 to 13 indicates an intermediate recovery stage in which the movement of the hind legs was recovered, but there was a gap in the harmonic motion in which the forelegs and hind legs were not well controlled. Finally, the scale of 14 to 21 indicates a late recovery stage in which forelegs and hind legs move harmonically.

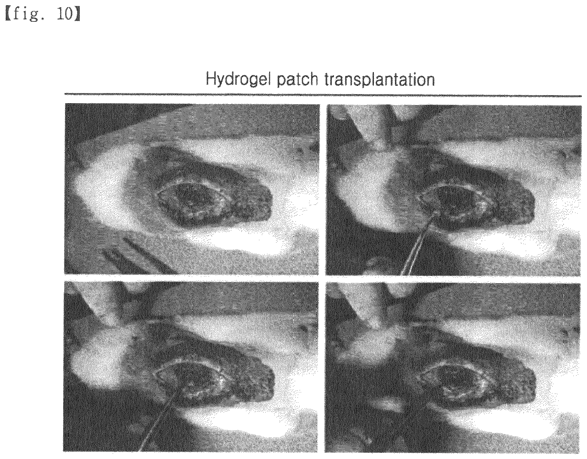

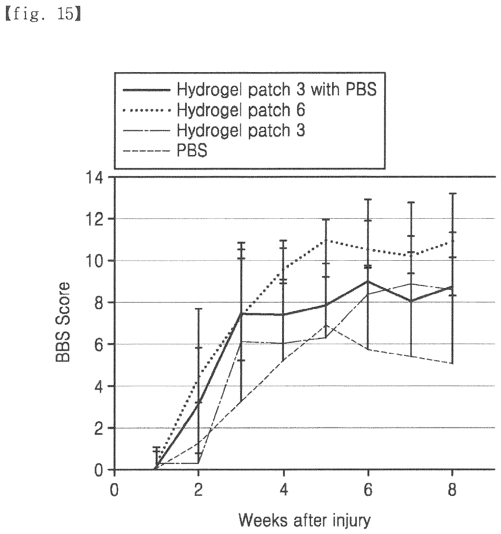

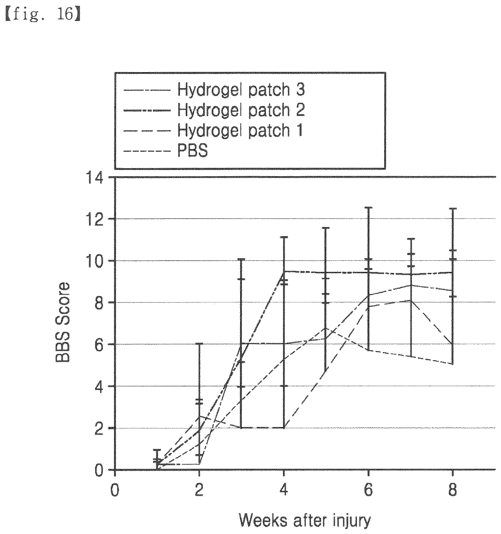

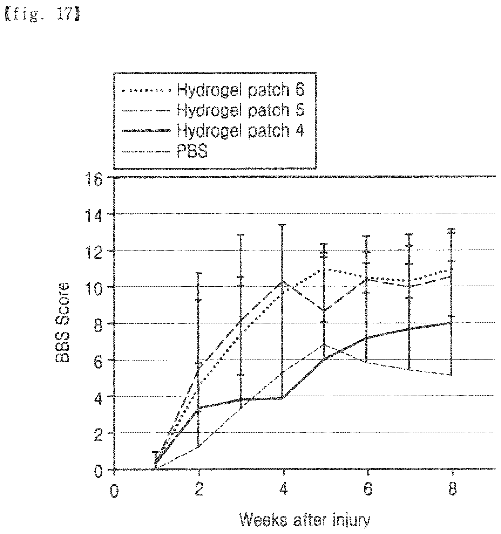

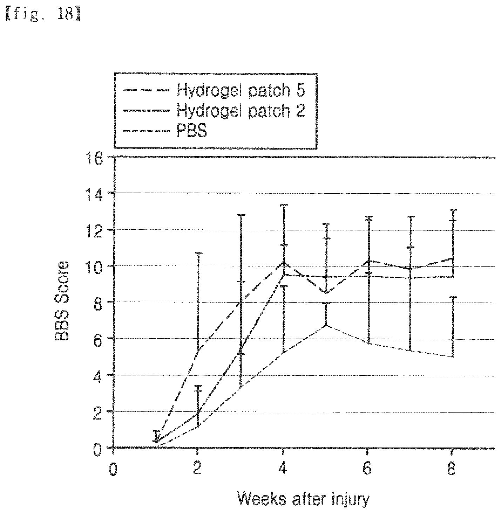

First, adult male Sprague-Dawley rats (OrientBio) was anesthetized by injection of 10 mg/rat Zoletil 50 (Virbac), and then, the spinal cord lamina of T9 site was removed therefrom. Then, the spinal cord was compressed for 10 minutes by using a vascular clip, thereby completing the preparation of a SCI animal model. After the SCI surgery, the bladder was compressed twice a day for about 2 weeks until the rates had voluntary urination. One week after the preparation of the SCI animal model, as shown in FIG. 10, a hydrogel patch according to an embodiment was directly transplanted in the SCI site. Behavioral evaluation was performed by using the SCI animal model evaluation method (BBB test; open field test) for 8 weeks, and the average of observation results of two observers the test results was used as test results, and the results are shown in FIGS. 15 to 18.

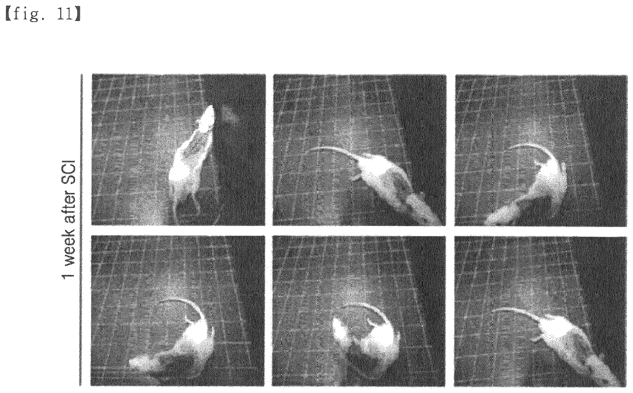

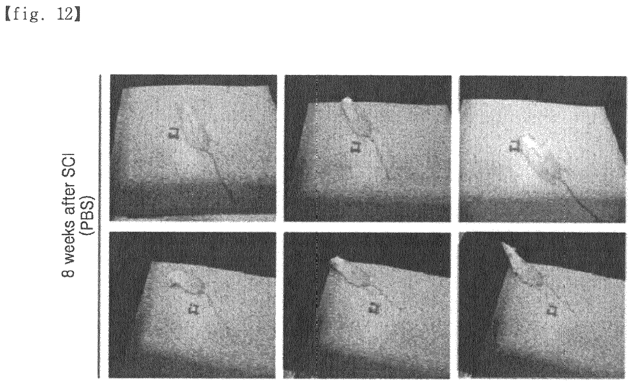

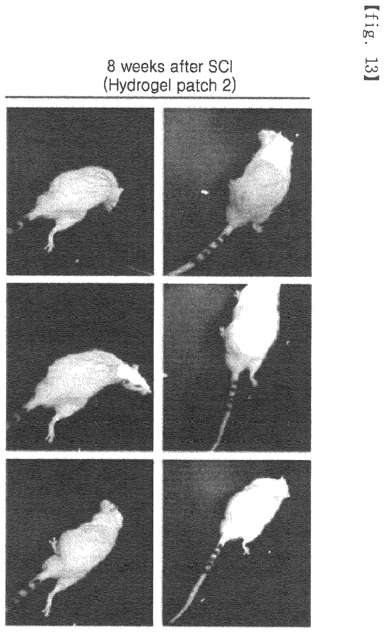

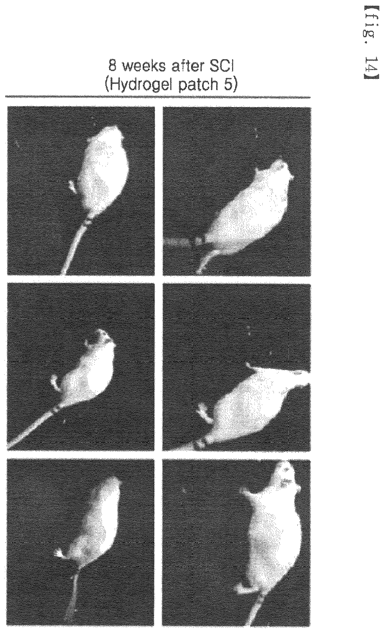

FIG. 11 shows an image of a SCI animal model to observe the movement of hind legs thereof, one week after the SCI. FIG. 12 shows an image of a SCI animal model to observe the movement of hind legs thereof, eight weeks after the SCI, when PBS alone, without the hydrogel patch, is applied on the transplantation site. FIG. 13 shows an image of a SCI animal model to observe the movement of hind legs thereof, eight weeks after the SCI, when hydrogel patch 2 (hydrogel patch 2 without GF) is transplanted. FIG. 14 shows an image of a SCI animal model to observe the movement of hind legs thereof, eight weeks after the SCI, when hydrogel patch 5 (hydrogel patch 2 with GF) is transplanted.

FIGS. 15 to 18 show BBB scores obtained according to transplantation of a hydrogel patch according to an embodiment.

As shown in FIGS. 11 to 14, it was confirmed that when PBS alone was applied on the transplantation site without the transplantation of the hydrogel patch, the SCI animal model showed no change in the motor ability of hind legs 8 weeks after the SCI. However, when hydrogel patch 2 was transplanted, the SCI animal model recovered its motor ability of a left hind leg 8 weeks after SCI, and when hydrogel patch 5 including a neuron growth factor and a vascular endothelial growth factor was planted, the SCI animal model remarkably recovered its motor ability of both hind legs 8 weeks after SCI.

In addition, the SCI recovery ability was evaluated by using a SCI animal model evaluation method (BBB test; open field test). The evaluation results show that, as illustrated in FIGS. 15 to 18, when PBS alone was applied on the transplantation site without the transplantation of the hydrogel patch, the SCI animal model had the BBB score of 4 to 6. This belongs to the early stage of the SCI regeneration process. However, when hydrogel patch 2 was transplanted, the SCI animal model had the BBB score of about 8 to about 10. This indicates an intermediate recovery stage of the SCI regeneration process. When hydrogel patch 5 was transplanted, the SCI animal model had the BBB score of about 10 to about 12. This indicates the late recovery state of the SCI regeneration process.

As illustrated in FIGS. 15 to 18, when hydrogel patch 1 or hydrogel patch 4 were transplanted, the SCI animal model had the BBB score of about 6 to about 8. This indicates the early stage of the SCI regeneration process. These results indicate that hydrogel patches containing fibrin, laminin, and a hyaluronic acid have a remarkable effect compared to other combinations.

In addition, when hydrogel patch 2 and hydrogel patch 5 were compared with hydrogel patch 3 and hydrogel patch 6 which include collagen, it was confirmed that there is no significant difference in the BBB scores.

In addition, hydrogel patch 3 was compared with the combination of hydrogel patch 3 and PBS, and it was found that there is no significant difference in the BBB scores. This result shows that a cell culture does not affect the efficacy of the hydrogel patch.

Therefore, it was confirmed that a hydrogel patch according to an embodiment has a therapeutic effect of remarkably recovering the motor ability lost by SCI.

### 4-2. Confirmation of treatment effects of hydrogel patch SCI by using histological analysis

The SCI regeneration effect of a hydrogel patch according to an embodiment was confirmed by histological analysis.

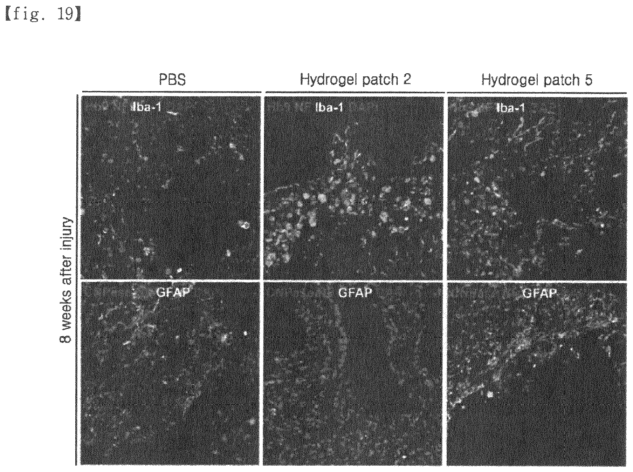

In detail, for histological analysis to identify the neuron regeneration effects of a hydrogel patch on the SCI site, rats were scarificed 8 weeks after the hydrogel patch transplantation surgery, and then, perfused by using 4% (w/v) paraformaldehyde (Merck) to collect a spinal cord. Thereafter, the spinal cord was cut to a thickness of 10 µm, and the cut spinal cord sample was stained in the same manner as in Experimental Example 1 by using anti-neurofilament (NF) (1:3000, Abeam), anti-GFAP (1:1000, Abcam), anti- 2',3'-Cyclic-nucleotide 3'-phosphodiesterase (CNPase) (1:800, Abcam), anti-homeobox HB9 (Hb9) (1:100, DSHB), and anti-ionized calciumbinding adapter molecule 1 (lba-1) (1:500,abeam). Thereafter, the hydrogel transplanted site was photographed by using a cofocal microscope (LSM 700, Zeiss) to evaluate the nerve regeneration. The results thereof are shown in FIG. 19.

As a result, as illustrated in FIG. 19, compared to when PBS was applied on the transplantation site, when hydrogel patch 2 or hydrogel patch 5 was transplanted, in SCI lesions, the distribution of GFAP astrocytes was low while the distribution of NF neurons was high. In addition, when the hydrogel patch 2 or hydrogel patch 5 was transplanted, the CNPase oligodendrocyte was co-localized at the same position as the NF neuron. These results show that due to the applying with hydrogel patch, the regeneration of injured neurons and the formation of myelin sheath of neurons were significantly increased.

Also, when PBS was applied on the transplantation site, the distribution of NF neurons was small and the distribution of lba-1 microglial cells, which show inflammatory response, was high. However, when hydrogel patch 2 or hydrogel patch 5 was transplanted, the distribution of motor neurons, which express Hb9 and NF of the spinal cord, was high, and the distribution of lba-1 microglial cells was substantially decreased. These results indicate that inflammatory responses, caused by the SCI when the hydrogel patch is transplanted, was reduced and thus the nerve injury was inhibited, and the regeneration of motor neurons and oligodendrocyte was promoted and the regeneration of the motor neuron with myelin sheath was promoted.

These results indicate that the hydrogel patch has substantially increased therapeutic effects of inducing the regeneration of injured spinal cord and inhibiting the activation of astrocytes to recover the lost motor ability, and the secondary SCI, caused by the SCI treatment using a syringe, may be substantially inhibited by using the hydrogel patch.

## Claims

1. A hydrogel patch or pharmaceutical composition for use in regenerating or covering an injured spinal cord tissue comprising:
at least one selected from fibrin and fibrinogen;
a concentration in a range of 2 µg/ml to 80 µg/ml of laminin; and
a concentration in a range of 10 µg/ml to 5 mg/ml of hyaluronic acid or a salt thereof;
wherein the hydrogel patch or pharmaceutical composition does not include cells.

2. The hydrogel patch or pharmaceutical composition for use of claim 1, further comprising a cell growth factor.

3. The hydrogel patch or pharmaceutical composition for use of claim 2, wherein the cell growth factor comprises a neuronal cell growth factor, a vascular endothelial cell growth factor, a fibroblast growth factor, a bone morphogenetic protein, an epidermal growth factor, a hepatocyte growth factor, a transforming growth factor, or a combination thereof.

4. The hydrogel patch for use of claim 1, wherein the hydrogel patch has a porous surface.

5. The hydrogel patch for use of claim 1, wherein the hydrogel patch is formed of a semi-solid, thereby a shape of the hydrogel patch conforms to a shape of an injured tissue site when the hydrogel patch is applied onto the injured tissue site.

6. The hydrogel patch or pharmaceutical composition for use of claim 1, wherein the concentration of fibrin or fibrinogen is in a range of 0.5 mg/ml to 20 mg/ml.

7. The hydrogel patch or pharmaceutical composition for use of claim 1, further comprising thrombin.

## Patentansprüche

1. Hydrogelpflaster oder pharmazeutische Zusammensetzung zur Verwendung bei der Regeneration oder zum Abdecken von verletztem Rückenmarksgewebe, Folgendes umfassend:
zumindest eines, ausgewählt aus Fibrin und Fibrinogen;
eine Konzentration von Laminin im Bereich von 2 µg/ml bis 80 µg/ml; und
eine Konzentration von Hyaluronsäure oder einem Salz davon im Bereich von 10 µg/ml bis 5 mg/ml;
wobei das Hydrogelpflaster oder die pharmazeutische Zusammensetzung keine Zellen enthält.

2. Hydrogelpflaster oder pharmazeutische Zusammensetzung zur Verwendung nach Anspruch 1, das weiters einen Zellwachstumsfaktor umfasst.

3. Hydrogelpflaster oder pharmazeutische Zusammensetzung zur Verwendung nach Anspruch 2, wobei der Zellwachstumsfaktor einen Nervenzellenwachstumsfaktor, einen Gefäßendothelwachstumsfaktor, einen Fibroblastenwachstumsfaktor, ein knochenmorphogenetisches Protein, einen Epidermiswachstumsfaktor, einen Hepatozytenwachstumsfaktor, einen transformierenden Wachstumsfaktor oder eine Kombination davon umfasst.

4. Hydrogelpflaster zur Verwendung nach Anspruch 1, wobei das Hydrogelpflaster eine poröse Oberfläche aufweist.

5. Hydrogelpflaster zur Verwendung nach Anspruch 1, wobei das Hydrogelpflaster aus einem Halbfeststoff gebildet ist, wodurch die Form des Hydrogelpflasters der Form einer verletzten Gewebestelle entspricht, wenn das Hydrogelpflaster auf die verletzte Gewebestelle aufgebracht wird.

6. Hydrogelpflaster oder pharmazeutische Zusammensetzung zur Verwendung nach Anspruch 1, wobei die Konzentration von Fibrin oder Fibrinogen im Bereich von 0,5 mg/ml bis 20 mg/ml liegt.

7. Hydrogelpflaster oder pharmazeutische Zusammensetzung zur Verwendung nach Anspruch 1, das weiters Thrombin umfasst.

## Revendications

1. Timbre d'hydrogel ou composition pharmaceutique à utiliser pour régénérer ou recouvrir un tissu de moelle épinière lésé, comprenant :
au moins un choisi parmi de la fibrine et du fibrinogène ;
une concentration dans une plage de 2 µg/ml à 80 µg/ml de laminine ; et
une concentration dans une plage de 10 µg/ml à 5 µg/ml d'acide hyaluronique ou d'un sel de celui-ci ;
le timbre d'hydrogel ou la composition pharmaceutique n'incluant pas de cellules.

2. Timbre d'hydrogel ou composition pharmaceutique à utiliser selon la revendication 1, comprenant en outre un facteur de croissance cellulaire.

3. Timbre d'hydrogel ou composition pharmaceutique à utiliser selon la revendication 2, le facteur de croissance cellulaire comprenant un facteur de croissance cellulaire neuronale, un facteur de croissance cellulaire endothéliale vasculaire, un facteur de croissance de fibroblastes, une protéine morphogénétique osseuse, un facteur de croissance épidermique, un facteur de croissance des hépatocytes, un facteur de croissance transformant, ou une combinaison de ceux-ci.

4. Timbre d'hydrogel à utiliser selon la revendication 1, dans lequel le timbre d'hydrogel présente une surface poreuse.

5. Timbre d'hydrogel à utiliser selon la revendication 1, dans lequel le timbre d'hydrogel est formé d'un semi-solide, moyennant quoi une forme du timbre d'hydrogel se conforme à une forme d'un site de tissu lésé lorsque le timbre d'hydrogel est appliqué sur le site de tissu lésé.

6. Timbre d'hydrogel ou composition pharmaceutique à utiliser selon la revendication 1, la concentration de fibrine ou de fibrinogène étant dans une plage de 0,5 mg/ml à 20 mg/ml.

7. Timbre d'hydrogel ou composition pharmaceutique à utiliser selon la revendication 1, comprenant en outre de la thrombine.
